# EUROPEAN PATENT SPECIFICATION

(11) **EP 1 797 904 B1**
(45) Date of publication and mention of the grant of the patent: **03.03.2010**
(21) Application number: 06076412.3
(22) Date of filing: 13.07.2006
(51) Int. Cl.: C07F 13/00, C01G 47/00, A61K 51/12, A61K 51/04

(54) **Method of preparing rhenium-188-tricarbonyl complex and its precursor**
Verfahren zur Herstellung des Tricarbonyltriaquakomplexes von Rhenium-188
Procédé de préparation de la complexe tricarbonyl triaqua de rhénium-188

(30) Priority: 16.12.2005 KR 20050124335
(43) Date of publication of application: 20.06.2007
(73) Proprietor: Korea Atomic Energy Research Institute, Daejeon 305-353 (KR)
(72) Inventor: Park, Sang Hyun, Daejeon 305-762 (KR); Byun, Myung Woo, Daejeon 305-333 (KR); Jang, Seung Ho, Daejeon 305-506 (KR)
(74) Representative: de Lang, Robbert-Jan

(56) References cited:
- WO-A-98/48848
- SCHIBLI R ET AL: "Steps toward high specific activity labelling of biomolecules for therapeutic application: preparation of precursor [[188]Re(H2O)3(CO)3]+ and synthesis of tailor-made bifunctional ligand systems" BIOCONJUGATE CHEMISTRY, vol. 13, no. 4, 7 July 2002 (2002-07-07), pages 750-756, XP002218738 ISSN: 1043-1802
- XIA J ET AL: "Characterization and application of the fac-[<188>Re(CO) 3(H2O)3]<+> core" JOURNAL OF RADIOANALYTICAL AND NUCLEAR CHEMISTRY, vol. 266, no. 2, 2005, pages 313-316, XP009075228 ISSN: 0236-5731
- SMITH C J ET AL: "Radiochemical investigations of [188Re(H2O)(CO)3-diaminopropionic acid-SSS-bombesin(7-14)NH2]: syntheses, radiolabeling and in vitro/in vivo GRP receptor targeting studies." ANTICANCER RESEARCH, vol. 23, no. 1A, January 2003 (2003-01), pages 63-70, XP009075236 ISSN: 0250-7005

## Description

### BACKGROUND OF THE INVENTION

### 1. Field of the Invention

The present invention relates to a method of preparing a ¹⁸⁸Re-tricarbonyl complex for radiopharmaceutical use and of preparing a precursor thereof, and more particularly, to a method of preparing a ¹⁸⁸Re-tricarbonyl precursor by mixing perrhenate with borane·ammonia (BH₃·NH₃), potassium boranocarbonate (K₂[H₃BCO₂]), and phosphate to react using borohydride exchange resin (BER) serving as a reducing agent, and to a method of preparing a ¹⁸⁸Re-tricarbonyl complex by reacting the ¹⁸⁸Re-tricarbonyl precursor with a ligand.

### 2. Description of the Related Art

In general, nuclear medicine technologies for using nuclear power in medicine definitely require the use of a radiopharmaceutical. As such, the radiopharmaceutical is prepared by selecting an appropriate material from among various kinds of radioisotopes generated when operating a nuclear reactor, then, processing it for use in the diagnosis or treatment of diseases and into a form able to be administered to the human body. Such a radiopharmaceutical can readily and obviously detect metastasis of cancer that is difficult or impossible to diagnose using other techniques.

When a diagnostic radiopharmaceutical is administered to the human body, it accumulates in specific internal organs of the body depending on the diagnostic purposes. Thereby, diseases occurring in various internal organs of the human body may be diagnosed. That is, when the radiopharmaceutical accumulates in the internal organs, such as the brain, bones, thyroid gland, heart, lungs, liver, spleen, kidney, etc., an image of the γ-rays emitted from the radiopharmaceutical accumulated in such internal organs can be obtained using a γ-camera. In addition to the internal organs, the radiopharmaceutical may also accumulate in cancer, inflammation, blood, etc.

Further, a therapeutic radiopharmaceutical is composed of radioactive radionuclides, which emit stronger radiation capable of killing cells despite the lower permeability of the human body and have a relatively longer half-life, compared to diagnostic radiopharmaceuticals. Such nuclides emit α-rays or β-rays. The nuclides emitting α-rays are highly toxic and are not readily available. Moreover, it is very difficult to label such radionuclides to materials other than diagnostic radionuclides. Thus, nuclides emitting β-rays have been used to date as radiopharmaceuticals.

An exemplary radioisotope widely used at present for labeling the diagnostic radiopharmaceutical is technetium-99m (^{99m}Tc). Since technetium has a relatively shorter half-life (6 hours) and emits only γ-ray energy (140 keV) suitable for obtaining a γ-image, it has low toxicity to the human body and high permeability therein, when administered to the human body to obtain a desired image.

In addition, rhenium, a homologue of technetium-99m, has preferable nuclear properties similar to those of technetium. For example, rhenium can emit energy usable for both diagnosis and therapy, and possesses a short half-life. Particularly, rhenium having isotopes of rhenium-186 (¹⁸⁶Re) and rhenium-188 (¹⁸⁸Re) can simultaneously emit β-rays, suitable for therapeutic application, and γ-rays for imaging. In practice, rhenium-186 or rhenium-188 has been used as a radiopharmaceutical applied to the treatment of bone pain that occurs due to secondary bone metastases of prostate cancer, lung cancer, breast cancer, etc. In addition, since rhenium-186 or rhenium-188 show chemical behaviors similar to those of technetium, it is possible to apply them to rhenium labeling methods via improvement in technetium labeling methods [ Lin, W. et al. Eur. J. Nucl. Med. 1997, 24, 590-595; Lewington, V. J. et al. Eur. J. Nucl. Med. 1993, 20, 66-74; Lewington, V. J. et al. Phys. Med. Biol. 1996, 41, 2027-2042; Hashimoto, K. et al. Appl. Radiat. Isot. 1996, 47, 195-199].

Typically, there have been proposed methods of preparing a technetium complex or a rhenium complex for use in a radiopharmaceutical comprising reacting the above metal with a ligand to form a complex and inducing a substitution reaction using another ligand, thereby labeling a target compound. Specifically, as a result of lyophilized glucoheptonate being subjected to a reaction with [^{99m}Tc] sodium pertechnate to prepare ^{99m}Tc-glucoheptonate, it was confirmed that ^{99m}Tc-glucoheptonate has an active site of [TcV=O]³⁺ [Owunwanne, A. et al, The Handbook of Radiopharmaceuticals, Chapman & Hall Medical, London, UK, p. 94-95].

Based on such a result, ^{99m}Tc-glucoheptonate is subjected to transchelation using a ligand that has greater affinity to technetium than to glucoheptonate that is the bonded ligand, and the peak of [TcV=O]³⁺ of the same species is identified through thin TLC or reverse phase HPLC, thereby proving the preparation of labeled technetium and determining the structure thereof.

In addition, attempts have been made to synthesize a ^{99m}TcNCl₄⁻ precursor by means of refluxing sodium azide (NaN₃) and pertechnetic acid or perrhenic acid in the presence of conc. hydrochloric acid to synthesize ^{99m}TcNCl_{4⁻} which is then subjected to a ligand substitution, thus obtaining [^{99m}TcV≡N]²⁺ [John Baldas and John Bonnyman Int. J. Appl. Radiot. Isot., 1985, 36, 133-139; Florian Demaimay, Leontine Dazord, Alain Roucoux, Nicolas Noiret, Herri Patin and Annick Moisan, Nuclear Medicine & Biology, 1997, 24, 701-705].

As mentioned above, in the process of forming the complex through the reaction between pertechnetic acid or perrhenic acid and the ligand, the reduction of technetium or rhenium should first be conducted. Such reduction may be carried out through electrolysis or may be performed using a reducing agent, including stannous chloride·dihydrate (SnCl₂·2H₂O), ferrous ion, ferrous-ascobate, formamidinesulfinic acid, or sodium borohydride. Generally, stannous chloride·dihydrate (SnCl₂·2H₂O) has been widely used.

However, the above-listed reducing agents have some drawbacks as follows; stannous chloride·dihydrate (SnCl₂·2H₂O) is stable under acidic conditions; whereas, it precipitates under basic conditions; and sodium borohydride is stable under basic conditions, whereas it is unstable under acidic conditions. In addition, when the above reducing agents in aqueous solution are excessively used, impurities such as colloids and the like may be produced, and furthermore, it is difficult to use the reducing agents in excess of predetermined amounts due to the problem of residual toxicity.

By contrast, other case have used the borohydride exchange resin, since the borohydride ion (BH₄⁻) bound to the exchange resin reacts in a solid phase instead of in aqueous solution, and may be filtered to remove it after the reaction, regardless of whether an excessive amount is applied to, thereby solving the above toxicity problem. Accordingly, numerous researches aimed at reducing pertechnetic acid or perrhenic acid under mild conditions in almost all pH ranges (pH 2 to 14) have continued to progress.

Recently, Alberto and his fellow researchers have reported the synthesis of a ^{99m}Tc-tricarbonyl complex having a low oxidation number of positive monovalence as a precursor for labeling biomolecules [Alberto R. et al., J. Am. Chem. Soc., 1998, 120, 7987-7988; Egli A. et al., J. Nucl. Med., 1999, 40(11), 1913-1917; Alberto R. et al., Radiochimica Acta., 1997, 79, 99-103; Alberto R. et al., J. Organometallic Chem., 1995, 493, 119-127; Reisgys M. et al., Bioorganic & Medicinal Chemistry Letters, 1997, 7(17), 2243-2246].

The above inventors have developed a convenient kit (IsoLinkTM) using potassium boranocarbonate (K₂[BH₃CO₂]) in order to prepare the ^{99m}Tc-tricarbonyl complex, in which the solid potassium boranocarbonate functions as a supply source of carbon monoxide and a reducing agent for reducing technetium.

With the intention of preparing the ¹⁸⁸Re-tricarbonyl complex, some research using the above method has been reported (Schibli, R., Schwarz, R., Alberto, R., Ortner, K., Schmalle, H., Dumas, C., Egli, A., and Schubiger, P. A. (2002) Steps toward high specific activity labeling of biomolecules for therapeutic application: preparation of precursor [188Re(OH2)3(CO)3]+ and synthesis of tailor-made bifunctional ligand systems. Bioconjugate Chem. 13, 750-756). As such, the above method is characterized in that potassium boranocarbonate is reacted with borane·ammonia in a neutral solution, thus reducing a perrhenic acid eluate. In order to prevent drastic hydrolysis of borane and to maintain a sufficiently low pH required for stabilization of a reduced rhenium intermediate, the amounts of reducing agent and acid (conc. phosphoric acid) should be cautiously controlled. As a result, the ¹⁸⁸Re-tricarbonyl complex has been reported to be synthesized in a yield of 85%.

Although the method provides the easy preparation of the ¹⁸⁸Re-tricarbonyl complex in a water phase, unreacted perrhenate ion ReO₄⁻ (7±3%), colloidal ¹⁸⁸ReO₂ (<5%), and unconfirmed by-product compositions may undesirably remain or may be produced. Therefore, there is the need for an improved method of preparing a ¹⁸⁸Re-tricarbonyl complex.

### SUMMARY OF THE INVENTION

Accordingly, the inventors of the present invention have carried out researches aimed at preventing the production of such undesirable by-products found in the existing art and obtaining a ¹⁸⁸Re-tricarbonyl precursor at a high yield by using borohydride exchange resin as a reducing agent and as an anion scavenger, and thereby completed the present invention.

An object of the present invention is to provide a novel method of preparing a ¹⁸⁸Re-tricarbonyl precursor, through which rhenium is labeled to a biomolecular ligand, thereby providing a method of preparing a ¹⁸⁸Re-tricarbonyl complex.

Another object of the present invention is to provide a contrast agent comprising the ¹⁸⁸Re-tricarbonyl complex.

In order to accomplish the above objects, the present invention provides, in a method of preparing a ¹⁸⁸Re-tricarbonyl precursor by mixing perrhenate with borane·ammonia (BH₃·NH₃), potassium boranocarbonate (K₂[H₃BCO₂]) and phosphate to react, a method of preparing a ¹⁸⁸Re-tricarbonyl precursor using borohydride exchange resin serving as a reducing agent, the method being represented by Scheme 1 below:

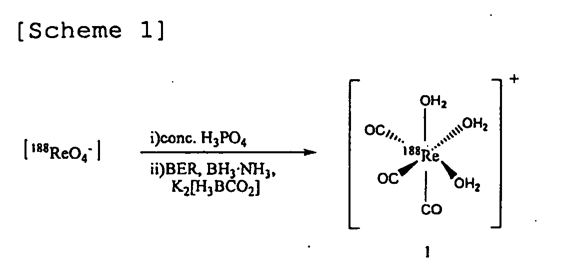

In addition, the present invention provides a method of preparing a ¹⁸⁸Re-tricarbonyl complex, comprising mixing and reacting perrhenate with borane·ammonia (BH₃·NH₃), potassium boranocarbonate (K₂[H₃BCO₂]) and phosphate in the presence of borohydride exchange resin serving as a reducing agent to prepare a ¹⁸⁸Re-tricarbonyl precursor; and reacting the ¹⁸⁸Re-tricarbonyl precursor with a ligand, to prepare a ¹⁸⁸Re-tricarbonyl complex, the method being represented by Scheme 2 below:

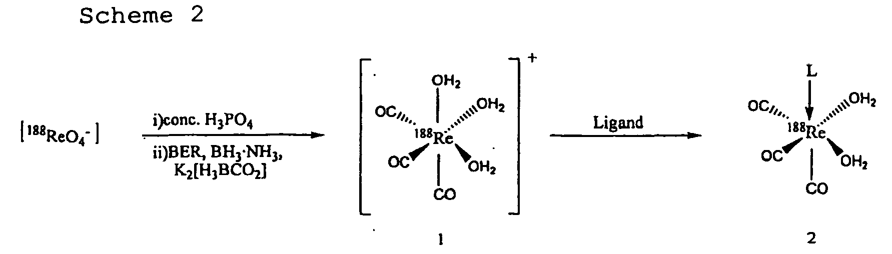

### BRIEF DESCRIPTION OF THE DRAWINGS

The above and other objects, features and advantages of the present invention will be more clearly understood from the following detailed description taken in conjunction with the accompanying drawings, in which:
FIG. 1 shows an HPLC chromatogram of ¹⁸⁸Re-tricarbonyl precursor and Na¹⁸⁸ReO₄ prepared in accordance with the present invention;
FIG. 2 shows an HPLC chromatogram of ^{99m}Tc-tricarbonyl precursor prepared in accordance with the present invention;
FIG. 3 shows a result of paper electrophoresis of ¹⁸⁸ Re-tricarbonyl precursor prepared in accordance with the present invention;
FIG. 4 shows an HPLC chromatogram of ¹⁸⁸Re-tricarbonyl histidine complex prepared in accordance with the present invention;
FIG. 5 shows an HPLC chromatogram of ^{99m}Tc-tricarbonyl histidine complex prepared in accordance with the present invention; and
FIG. 6 shows a schematic view of borohydride exchange resin used as a solid-phase reducing agent and as an anion scavenger.

### DESCRIPTION OF THE PREFERRED EMBODIMENTS

Hereinafter, a detailed description of the present invention will be given.

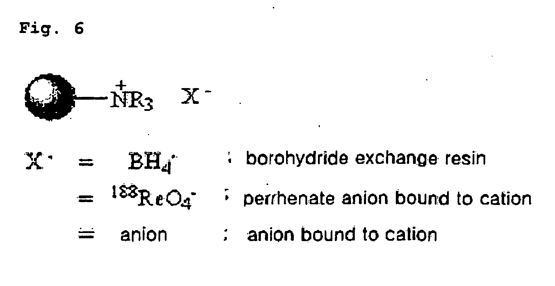

In the' method of preparing the ¹⁸⁸Re-tricarbonyl precursor (1) of the present invention, borohydride exchange resin functions as a reducing agent for reducing perrhenate. The borohydride exchange resin is structured in such a manner that a borohydride ion (BH₄⁻) is coupled with a cation supported to the exchange resin, the cation being usable for fixation of the borohydride ion including, for example, quaternary ammonium functionality as depicted in FIG. 6, and being used in an amount suitable for reducing perrhenic acid sufficiently.

As an exchange resin to which the borohydride ion may be supported, any anion exchange resin such as polystyrene, high-density polyethylene, amberite and the like may be used so long as it has quaternary ammonium functionality.

In addition, since the borohydride exchange resin is stable in almost all pH ranges (pH 2 to 11) it can be readily applied to biomolecules.

Further, the borohydride exchange resin functions as an anion scavenger. As a result, the unreacted perrhenate anion ReO₄⁻, negatively charged impurities and the like are captured by the borohydride exchange resin and easily removed through a subsequent filtration process, thereby effectively preparing a highly pure ¹⁸⁸Re-tricarbonyl precursor.

The borohydride exchange resin is preferably used in an amount of 3~5 mg, based on 50 MBq of sodium perrhenate, in order to effectively reduce perrhenic acid and sufficiently function as an anion scavenger. If the amount of borohydride exchange resin is less than 3 mg, it is too small to scavenge the anions. On the other hand, if the amount exceeds 5 mg, the borohydride exchange resin is undesirably wasted.

In the method of preparing the ¹⁸⁸Re-tricarbonyl precursor of the present invention, potassium boranocarbonate functions as a source for supplying carbon monoxide and also as a reducing agent for reducing rhenium. Such potassium boranocarbonate is preferably used in an amount of 3~4 mg, based on 50 MBq of sodium perrhenate, so as to realize sufficient chelation to rhenium. If the amount of potassium boranocarbonate is less than 3 mg, it is difficult to conduct a sufficient chelation. On the other hand, if the amount exceeds 4 mg, the reagent is undesirably wasted.

In the method of preparing the ¹⁸⁸Re-tricarbonyl precursor of the present invention, borane·ammonia functions as a reducing agent for reducing rhenium. Such borane·ammonia is preferably used in an amount of 3~4 mg, based on 50 MBq of sodium perrhenate. If the amount of potassium boranocarbonate used falls outside the above range, the reduction occurs insufficiently or the reagent is undesirably wasted.

In the method of preparing the ¹⁸⁸Re-tricarbonyl precursor of the present invention, in addition to the borohydride exchange resin, potassium boranocarbonate and borane·ammonia may be used together as the reducing agent. This is because rhenium is strongly bound due to its higher electron density differently from technetium.

In the method of preparing the ¹⁸⁸Re-tricarbonyl precursor of the present invention, phosphoric acid stabilizes a rhenium intermediate reduced in the presence of the reducing agents. For this, since the pH should be sufficiently decreased, it is desired to use conc. phosphoric acid of 85% or more.

In the method of preparing the ¹⁸⁸Re-tricarbonyl precursor of the present invention, the reduction of perrhenic acid and the chelation are preferably conducted at 55∼65°C for 10~20 minutes so as to effectively reduce perrhenic acid and chelate carbon monoxide. After completing the reduction of perrhenic acid under the above reaction conditions, the resulting ¹⁸⁸Re-tricarbonyl precursor is preferably cooled to room temperature for stabilization.

Further, the present invention provides a method of preparing a ¹⁸⁸Re-tricarbonyl complex (2) comprising preparing the ¹⁸⁸Re-tricarbonyl precursor and chelating the prepared ¹⁸⁸Re-tricarbonyl precursor with a ligand.

In the method of preparing the ¹⁸⁸Re-tricarbonyl complex of the present invention, the ligand to be chelated to the ¹⁸⁸Re-tricarbonyl precursor may be of any type so long as it forms a complex along with rhenium. In the case where the complex is formed, it is desired to use a ligand having a coordinate number of 1, 2 or 4 in order to stabilize rhenium stereochemically.

The functional group of the ligand includes amine, carboxyl, thiolate, nitrido, isocyanate, alcohol, ester, halogen elements, alkoxy, sulfonic acid, nitro, amide, nitrile, isonitrile, etc. For example, the ligand is one selected from the group consisting of nitrido, glucoheptonate, L-cysteine, L-cysteine-hydrochloric acid·water, histidine, diaminedisulfide, dimercaptosuccinic acid, thio-β-D-glucose, methylene diphosphate, diethylenetriaminepentaacetic acid, and N-[2-(2-((triphenylmethyl)thio)ethyl)acetyl]-S-(triphenylmethyl)-2-aminoethanethiol.

In addition, since the borohydride exchange resin used as the reducing agent of the present invention is stable in almost all pH ranges, any biomolecule may be directly applied so long as it has the above ligand functionality. For example, it is preferable to use any biomolecule if it can readily form a peptide via an amide bond resulting from combination with an amino acid, which is a component of protein that is a major constituent of the human body. Such a biomolecular ligand may be selected from the group consisting of human serum albumin, peptide, and human immune globulin.

In the method of preparing the ¹⁸⁸Re-tricarbonyl complex of the present invention, the reaction for chelating the ligand to the ¹⁸⁸Re-tricarbonyl precursor is preferably conducted at 70~80°C for 25~35 minutes for effective chelation between the ligand and the rhenium-tricarbonyl precursor. After completing the chelation under the above reaction conditions, the resulting rhenium-tricarbonyl-biomolecule complex is cooled to room temperature for stabilization.

As a solvent required for synthesizing the ¹⁸⁸Re-tricarbonyl precursor and the ¹⁸⁸Re-tricarbonyl complex, an aqueous solvent, for example, water, acetone, methanol, ethanol, or mixtures thereof, may be used.

In addition, the present invention provides a contrast agent comprising the ¹⁸⁸Re-tricarbonyl-biomolecule complex composed of the ¹⁸⁸Re-tricarbonyl precursor and the biomolecule as the ligand bound thereto.

The contrast agent of the present invention may be provided as the ¹⁸⁸Re-tricarbonyl-biomolecule complex alone or in kit form including the ¹⁸⁸Re-tricarbonyl-biomolecule complex. Since the contrast agent includes an aqueous saline medium, as well as the ¹⁸⁸Re-tricarbonyl complex, it may be administered via intravenous injection. The medium includes a pharmaceutically acceptable salt, a buffer solution, or a medical adjuvant typically used, such as antiseptic.

A better understanding of the present invention may be obtained in light of the following examples which are set forth to illustrate, but are not to be construed to limit the present invention.

### <Embodiment 1> Synthesis of Rhenium-Tricarbonyl Precursor

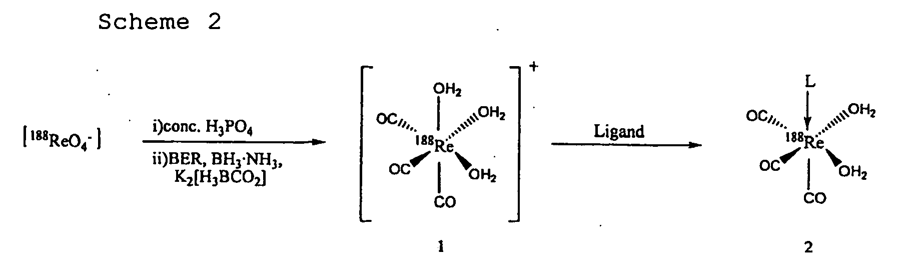

3 mg of borane·ammonia, 3 mg of potassium boranocarbonate, and 3 mg of borohydride exchange resin were placed in a 10 ml vial, which was then sealed with a rubber plug. 1 ml of about 50 MBq sodium perrhenate solution and 7 µl of conc. phosphoric acid (85%) were injected into the vial using a syringe. Subsequently, the resulting mixture was subjected to react at 60°C for 15 minutes using a boiling water bath, and the resulting reactant was cooled to room temperature, thus obtaining a neutral ¹⁸⁸Re-tricarbonyl precursor (1) in a yield of 97% or more.

### <Embodiment 2> Synthesis of ¹⁸⁸Re (I)-Tricarbonyl Histidine Complex

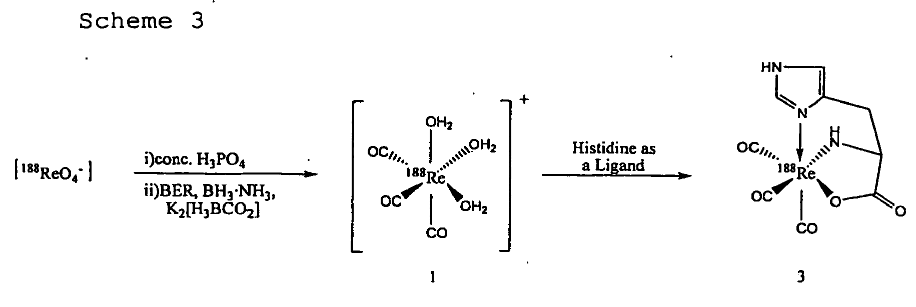

500 µl of histidine was placed in a 10 ml vial, which was then sealed with a rubber plug. Into the vial, 800 µl of the 50 MPq ¹⁸⁸Re (I)-tricarbonyl precursor solution synthesized in Embodiment 1 was injected using a syringe. Subsequently, the vial was heated to 75°C for 30 minutes and then cooled to room temperature, thus obtaining a ¹⁸⁸Re (I)-tricarbonyl histidine complex (3) in a yield of 97% or more.

### <Experimental Example 1> HPLC Measurement of ¹⁸⁸Re (I)-Tricarbonyl Precursor

The HPLC measurement was conducted in order to separate the ¹⁸⁸Re (I)-tricarbonyl precursor synthesized in Embodiment 1.

The HPLC measurement was carried out with a WATERS system provided with a radiometric detector using methanol (hereinafter, referred to as 'solvent A') and 0.05 M triethylammoniumphosphate buffer (TEAP, pH 2.25, hereinafter referred to as 'solvent B') using two pumps each equipped with a reverse phase XterraTM RP18 5 µm column (4.6 x 250 mm, Waters, Ireland).

The solvent conditions for HPLC were as follows: in the range of 0 to 5 minutes, 100% solvent A began to flow and was then converted into 100% solvent B; in the range of 5 to 8 minutes, 100% solvent B began to flow and was then converted into 25% solvent A and 75% solvent B; in the range of 8 to 11 minutes, 25% solvent A and 75% solvent B were converted into 34% solvent A and 66% solvent B; in the range of 11 to 22 minutes, 34% solvent A and 66% solvent B were converted into 100% solvent A; and in the range of 22 to 24 minutes, 100% solvent A was converted into 100% solvent B. The flow rate of solvent was maintained at 1 ml/min for the entire time. In addition, in order to rapidly bring the column to a state of equilibrium, during the next 5 min, the solvent was supplied at 2 ml/min for 2 minnutes and then at the former solvent flow rate for 3 min.

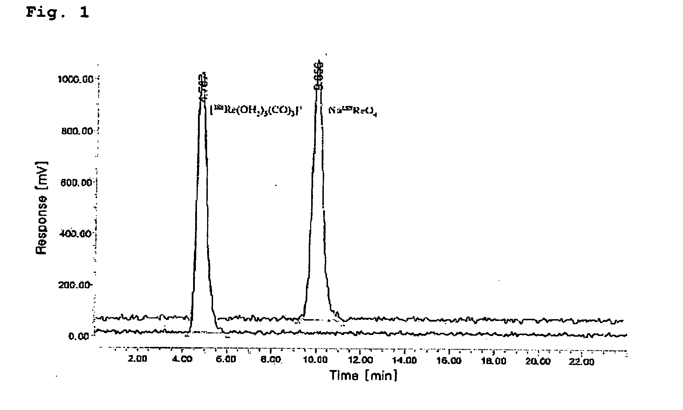

The result of HPLC analysis is shown as an HPLC chromatogram in FIG. 1. As shown in FIG. 1, the ¹⁸⁸Re (I)-tricarbonyl precursor and the perrhenate anion ReO₄⁻ had retention times of 4.7 minutes and 9.8 minutes, respectively. The radiolabeling yield was measured to be 97% or more.

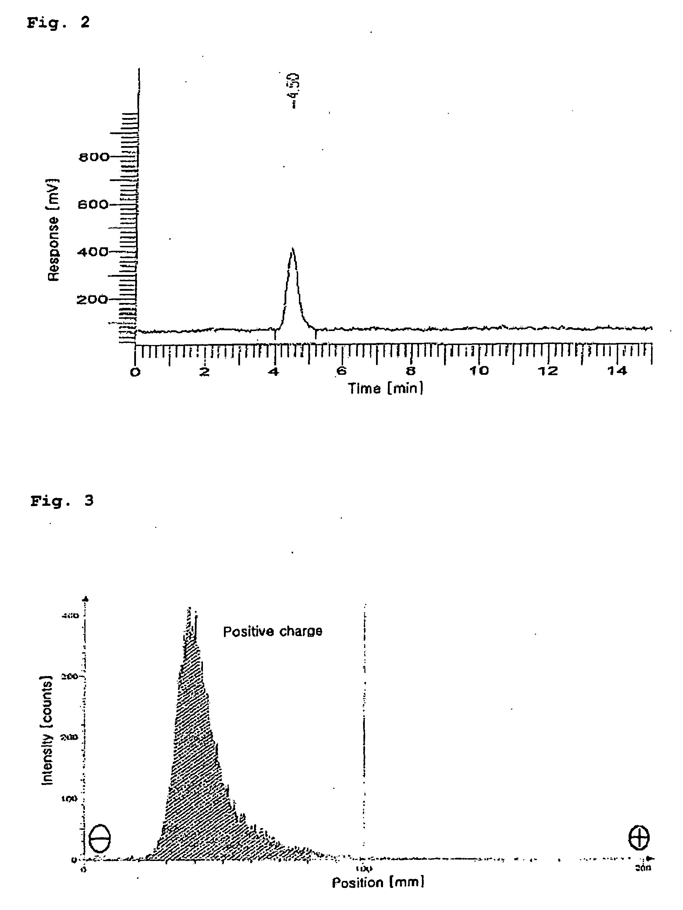

The retention time of ¹⁸⁸Re (I)-tricarbonyl precursor was proven to be similar to that of ^{99m}Tc(I)-tricarbonyl precursor, mainly used as a radiolabeled pharmaceutical (FIG. 2).

95% or more of the ¹⁸⁸Re (I)-tricarbonyl precursor was stable for 3 hours and then began to decay. As shown in FIG. 3, the positive charge of the ¹⁸⁸Re (I)-tricarbonyl precursor present in the neutral solution could be confirmed through paper electrophoresis in the aqueous solution.

The ¹⁸⁸Re(I)-tricarbonyl precursor, the reduced hydrolyzed ¹⁸⁸Re, and the perrhenate ion were analyzed by observing the positions thereof using ITLC (Instant Thin Layer Chromatography):
¹⁸⁸Re(I)-tricarbonyl precursor: > 95% (R_{f} = 0.4);
Reduced hydrolyzed ¹⁸⁸Re: 3% or less (origin); and
Perrhenate ion: 0% (R_{f} = 0.8).

### <Experimental Example 2>

### HPLC Measurement of ¹⁸⁸Re(I)-Tricarbonyl Histidine Complex

In order to confirm the ¹⁸⁸Re(I)-tricarbonyl histidine complex prepared in Embodiment 2, the HPLC measurement was conducted.

The HPLC measurement was carried out with a Perkin Elmer system provided with a radiometric detector (IsoScan LC gamma, Biostep, Germany) using a Hypersil ODS column (filler 10 µm, 250 x 4 mm, KNAUER, Berlin, Germany) using ethanol (solvent A) and 0.05 M TEAP buffer (pH 1.95, solvent B) as HPLC solvents.

The solvent conditions for HPLC were as follows: in the range of 0 to 10 minutes, 100% solvent A began to flow and was then converted into 100% solvent B; in the range of 10 to 20 minutes, only 100% solvent A was supplied; and in the range of 20 to 25 minutes, 100% solvent A was converted into 100% solvent B. The flow rate of solvent was maintained at 1 ml/min for the entire time. In addition, all of the solvents used as a mobile phase corresponding to the HPLC grade were pre-filtered via a bottle filter having a pore size of 0.2 µm.

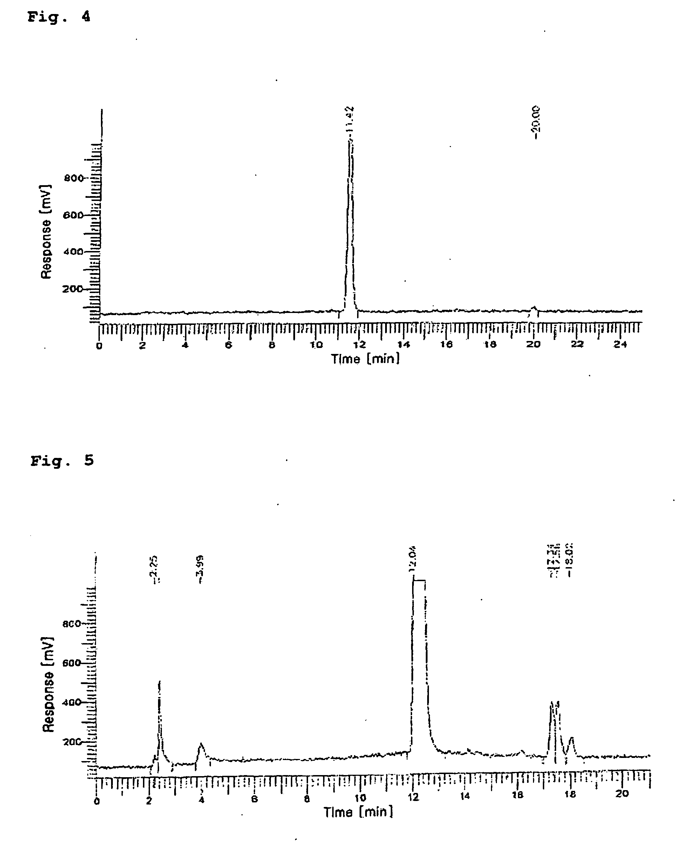

The result of the HPLC measurement is shown as an HPLC chromatogram in FIG. 4. As shown in FIG. 4, the retention time of ¹⁸⁸Re(I)-tricarbonyl histidine complex was 11.4 min. The radiolabeling yield was measured to be 97% or more.

The retention time of the ¹⁸⁸Re(I)-tricarbonyl histidine complex was proven to be similar to that of ^{99m}Tc (I) -tricarbonyl histidine, mainly used as a radiolabeled pharmaceutical (FIG. 5).

### <Experimental Example 3>

### Test of Plasma Stability of ¹⁸⁸Re-Tricarbonyl Histidine Complex

The ¹⁸⁸Re-tricarbonyl histidine complex prepared in Embodiment 2 was dissolved in physiological saline to prepare 37 MBq/ml of a test solution. Of this solution, 25 µl of the solution was added to 475 µl of human plasma (sigma) and then cultured at 37°C for 24 hours. After the lapse of 0.5, 1, 2, 4 and 24 hours, the fraction of solution was subjected to TLC measurement. As a development solvent, a solvent mixture comprising methanol and conc. hydrochloric acid mixed at a 99:1 ratio was used.

When the fraction of solution was developed, the labeled complex was shown in the developed portion, and the complex reacted with the plasma protein was shown in the zero point, which were confirmed through HPLC. The HPLC measurement was conducted in the same manner as in Experimental Example 2. In addition, the plasma protein binding was measured using an ITLC isotope scanner. The results are given in Table 1 below.

**TABLE 1**

| Time (hr) | Plasma Protein Binding (%) |
|---|---|
| 0.5 | 61.4 |
| 1 | 68.0 |
| 2 | 73.9 |
| 6 | 74.8 |

As will be apparent from Table 1, the ¹⁸⁸Re-tricarbonyl histidine complex reacted with the plasma protein was merely 61% at 0.5 hours, and was then maintained at 75% or less after the lapse of 6 hours. Thus, the complex of the present invention can maintain high plasma stability and therefore can be usefully applied to a contrast agent.

As described hereinbefore, the present invention provides a method of preparing a ¹⁸⁸Re-tricarbonyl precursor and complex. According to the method of the present invention, borohydride exchange resin is used as a reducing agent and as an anion scavenger, thereby obtaining the ¹⁸⁸Re-tricarbonyl precursor and complex having high radiolabeling yield and high purity. In addition, the ¹⁸⁸Re-tricarbonyl complex can be used as a contrast agent having excellent plasma stability.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. In a method of preparing a ¹⁸⁸Re-tricarbonyl precursor by mixing perrhenate with borne-ammonia (BH₃·NH₃), potassium boranocarbonate (K₂ [H₃BCO₂]) and phosphate to react, a method of preparing a ¹⁸⁸Re-tricarbonyl precursor using borohydride exchange resin serving as a reducing agent, the method being represented by Scheme 1 below:

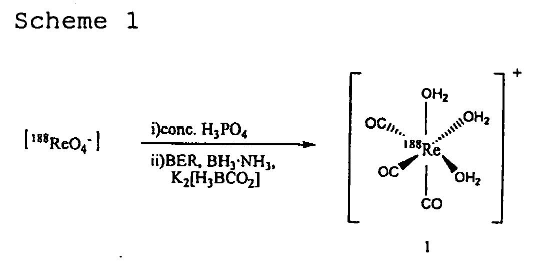

2. The method as set forth in claim 1, wherein the reaction to prepare the ¹⁸⁸Re-tricarbonyl precursor is conducted at 55~65°C for 10~20 min.

3. The method as set forth in claim 1, wherein the borohydride exchange resin is used in an amount of 3~5 mg, based on 50 MBq of sodium perrhenate.

4. The method as set forth in claim 1, wherein the potassium boranocarbonate is used in an amount of 3~4 mg, based on 50 MBq of sodium perrhenate.

5. The method as set forth in claim 1, wherein the borane·ammonia is used in an amount of 3~4 mg, based on 50 MBq of sodium perrhenate.

6. A method of preparing a ¹⁸⁸Re-tricarbonyl complex, comprising:
mixing and reacting perrhenate with borane·ammonia (BH₃·NH₃), potassium boranocarbonate (K₂[H₃BCO₂]) and phosphate in the presence of borohydride exchange resin serving as a reducing agent to prepare a ¹⁸⁸Re-tricarbonyl precursor; and
reacting the ¹⁸⁸Re-tricarbonyl precursor with a ligand to prepare a ¹⁸⁸Re-tricarbonyl complex, the method being represented by Scheme 2 below:

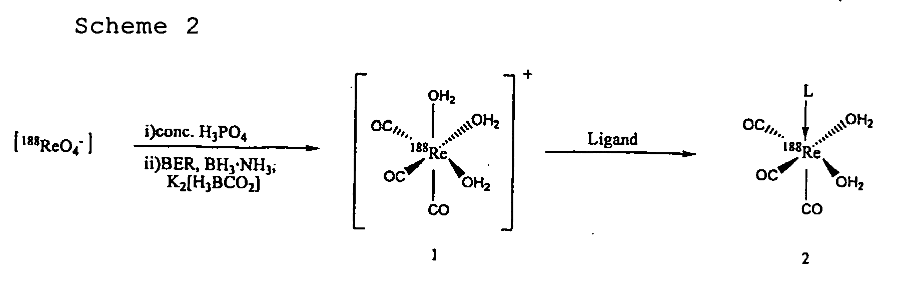

7. The method as set forth in claim 6, wherein the ligand is any one selected from the group consisting of nitrido, glucoheptonate, L-cysteine, L-cysteine·hydrochloric acid·water, histidine, diaminedisulfide, dimercaptosuccinic acid, thio-β-D-glucose, methylene diphosphate, diethylenetriaminepentaacetic acid, and N-[2-(2-((triphenylmethyl)thio)ethyl)acetyl]-S-(triphenylmethyl)-2-aminoethanethiol.

8. The method as set forth in claim 6, wherein the reaction to prepare the ¹⁸⁸Re-tricarbonyl complex is conducted at 70~80°C for 25~35 min.

## Patentansprüche

1. Verfahren zum Herstellen einer ¹⁸⁸Re-Tricarbonyl-Vorstufe durch Vermischen von Perrhenat mit Boran·Ammoniak (BH₃·NH₃), Kaliumboranocarbonat (K₂[H₃BCO₂]) und Phosphat unter Verwendung eines Borhydrid-Austauschharzes, das als Reduktionsmittel dient, wobei das Verfahren durch nachstehendes Schema 1 wiedergegeben ist:

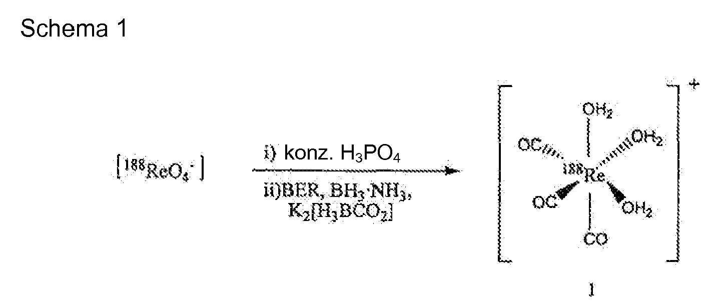

2. Verfahren nach Anspruch 1, wobei die Reaktion zum Herstellen der ¹⁸⁸Re-Tricarbonyl-Vorstufe bei 55~65 °C während 10~20 min durchgeführt wird.

3. Verfahren nach Anspruch 1, wobei das Borhydrid-Austauschharz in einer Menge von 3~5 mg, bezogen auf 50 MBq Natriumperrhenat, verwendet wird.

4. Verfahren nach Anspruch 1, wobei das Kaliumboranocarbonat in einer Menge von 3~4 mg, bezogen auf 50 MBq Natriumperrhenat, verwendet wird.

5. Verfahren nach Anspruch 1, wobei das Boran·Ammoniak in einer Menge von 3~4 mg, bezogen auf 50 MBq Natriumperrhenat, verwendet wird.

6. Verfahren zum Herstellen eines ¹⁸⁸Re-Tricarbonyl-Komplexes, umfassend:
Vermischen und Umsetzen von Perrhenat mit Boran·Ammoniak (BH₃·NH₃), Kaliumboranocarbonat (K₂[H₃BCO₂]) und Phosphat in Gegenwart eines Borhydrid-Austauschharzes, das als Reduktionsmittel dient, um eine ¹⁸⁸Re-Tricarbonyl-Vorstufe herzustellen; und
Umsetzen der ¹⁸⁸Re-Tricarbonyl-Vorstufe mit einem Liganden, um einen ¹⁸⁸Re-Tricarbonyl-Komplex herzustellen, wobei das Verfahren durch nachstehendes Schema 2 wiedergegeben ist:

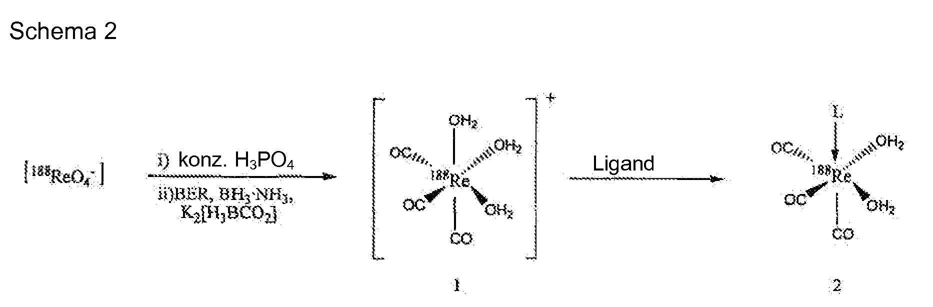

7. Verfahren nach Anspruch 6, wobei der Ligand ein beliebiger Ligand, ausgewählt aus der Gruppe bestehend aus Nitrido, Glucoheptonat, L-Cystein, L-Cystein·Chlorwasserstoffsäure·Wasser, Histidin, Diamindisulfid, Dimercaptobernsteinsäure, Thio-β-D-Glucose, Methylendiphosphat, Diethylentriaminpentaessigsäure und N-[2-(2-((Triphenylmethyl)thio)ethyl)acetyl]-S-(triphenylmethyl)-2-aminoethanthiol, ist.

8. Verfahren nach Anspruch 6, wobei die Reaktion zum Herstellen des ¹⁸⁸Re-Tricarbonyl-Komplexes bei 70~80 °C während 25~35 min durchgeführt wird.

## Revendications

1. Dans un procédé de préparation d'un précurseur de ¹⁸⁸Re-tricarbonyle par mélange d'un perrhénate avec du borane-ammoniac (BH₃-NH₃), du boranocarbonate de potassium (K₂[H₃BCO₂]) et du phosphate pour qu'ils réagissent, procédé de préparation d'un précurseur de ¹⁸⁸Re-tricarbonyle utilisant une résine d'échange de borohydrure servant d'agent réducteur, le procédé étant représenté par le Schéma 1 ci-dessous :

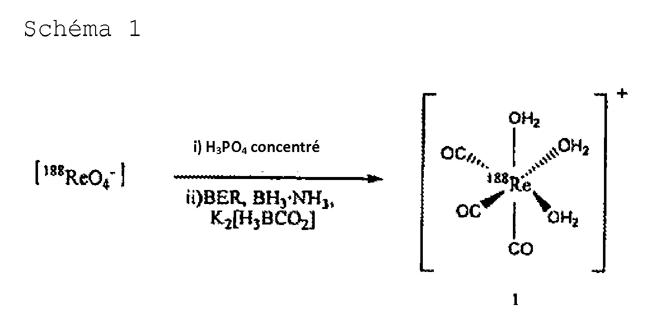

2. Procédé selon la revendication 1, dans lequel la réaction pour préparer le précurseur de ¹⁸⁸Re-tricarbonyle est conduite à 55 ~ 65°C pendant 10 ∼ 20 min.

3. Procédé selon la revendication 1, dans lequel la résine d'échange de borohydrure est utilisée dans une quantité de 3 ∼ 5 mg, sur la base de 50 MBq de perrhénate de sodium.

4. Procédé selon la revendication 1, dans lequel le boranocarbonate de potassium est utilisé dans une quantité de 3 ∼ 4 mg, sur la base de 50 MBq de perrhénate de sodium.

5. Procédé selon la revendication 1, dans lequel le complexe de borane-ammoniac est utilisé dans une quantité de 3 - 4 mg, sur la base de 50 MBq de perrhénate de sodium.

6. Procédé de préparation d'un complexe de ¹⁸⁸Re-tricarbonyle, comprenant :
le mélange et la réaction d'un perrhénate avec du borane-ammoniac (BH₃-NH₃), du boranocarbonate de potassium (K₂[H₃BCO₂]) et du phosphate en présence d'une résine d'échange de borohydrure servant d'agent réducteur pour préparer un précurseur de ¹⁸⁸Re-tricarbonyle ; et
la réaction du précurseur de ¹⁸⁸Re-tricarbonyle avec un ligand pour préparer un complexe de ¹⁸⁸Re-tricarbonyle, le procédé étant représenté par le Schéma 2 ci-dessous :

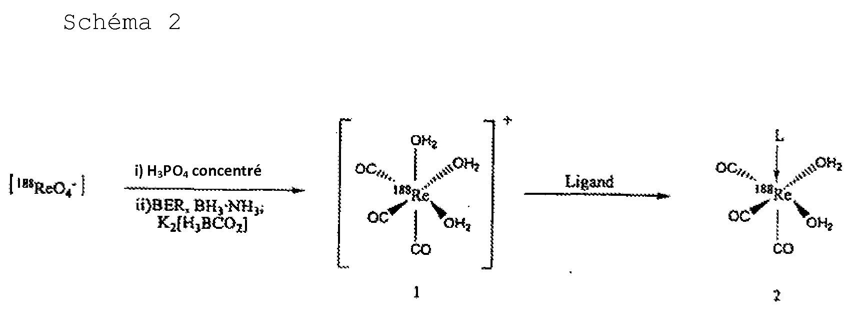

7. Procédé selon la revendication 6, dans lequel le ligand est un quelconque ligand choisi parmi le groupe consistant en un nitrido, un glucoheptonate, la L-cystéine, un complexe de L-cystéine-acide chlorhydrique-eau, l'histidine, le disulfure de diamine, l'acide dimercaptosuccinique, le thio-β-D-glucose, le diphosphate de méthylène, l'acide diéthylènetriaminepentaacétique, et le N-[2-(2-((triphénylméthyl)thio)éthyl)acétyl]-S-(triphénylméthyl)-2-aminoéthanethiol.

8. Procédé selon la revendication 6, dans lequel la réaction pour préparer le complexe de ¹⁸⁸Re-tricarbonyle est conduite à 70 ∼ 80°C pendant 25 ~ 35 min.
